Europäisches Patentamt

European Patent Office

Office européen des brevets

(11) Publication number: **0 092 933**
**B1**

# EUROPEAN PATENT SPECIFICATION

(45) Date of publication of patent specification: **11.12.85**

(51) Int. Cl.⁴: **C 07 K 5/06**

(21) Application number: **83302061.3**

(22) Date of filing: **13.04.83**

(54) **Process for producing alpha-L-aspartyl-L-phenylalanine methyl ester or its hydrochloride.**

(30) Priority: **22.04.82 JP 67687/82**

(43) Date of publication of application:
**02.11.83 Bulletin 83/44**

(45) Publication of the grant of the patent:
**11.12.85 Bulletin 85/50**

(84) Designated Contracting States:
**BE CH DE FR GB IT LI NL**

(56) References cited:
**EP-A-0 058 063**
**US-A-3 933 781**
**US-A-4 173 562**

(73) Proprietor: **AJINOMOTO CO., INC.**
**5-8, Kyobashi 1-chome, Chuo-ku**
**Tokyo 104 (JP)**

(72) Inventor: **Takemoto, Tadashi**
**No. 1155-2, Nakamaruko Nakahara-ku**
**Kawasaki-shi Kanagawa-ken (JP)**
Inventor: **Ariyoshi, Yasuo**
**No. 4-20-14, Ooka Minami-ku**
**Yokohama-shi Kanagawa-ken (JP)**

(74) Representative: **Bond, Bentley George et al**
**Haseltine Lake & Co. 28 Southampton Buildings**
**Chancery Lane**
**London WC2A 1AT (GB)**

EP 0 092 933 B1

Courier Press, Leamington Spa, England.

## Description

This invention relates to a process for producing α - L - aspartyl - L - phenylalanine methyl ester (referred to herein as "α-APM") or its hydrochloride, more particularly to a process for producing α-APM or its hydrochloride which comprises (a) removing the formyl group efficiently and selectively from α-APM whose amino group is protected with a formyl group, i.e. from N - formyl - α - L - aspartyl - L - phenylalanine methyl ester (referred to herein as "For-α-APM", in a methanol-hydrochloric acid mixture, i.e. in methanolic hydrochloric acid; (b) separating the formed α-APM and its hydrochloride, i.e. as α - L - aspartyl - L - phenylalanine methyl ester hydrochloride (referred to herein as "α-APM.HCl") and (c) converting the separated α-APM.HCl, if desired, to free α-APM. In this connection, the conversion of α-APM.HCl to α-APM may be carried out by a conventional method.

This invention provides a useful means of synthesizing a dipeptide sweetener and more specifically an efficient method of removing the formyl group from For-α-APM.

For-α-APM, as an intermediate for the synthesis of α-APM, may be easily produced by condensation between N - formyl - L - aspartic acid anhydride and L-phenylalanine methyl ester (see JP—A—23001/1977, which corresponds to US—A—4 071 511).

It is known that an N-formyl group, i.e. a formyl group protecting an amino group, can be removed by the use of dilute hydrochloric acid, of hydrogen peroxide (G. Losse *et al.*, Ann. Chem., *636*, 140 (1960)), of hydrazine (P. Lefrancier *et al.*, Bull. Soc. Chim. France, *1965*, 3668), of aniline (R. Geiger *et al.*, Chem. Ber., *102*, 2487(1969)) or of hydroxylamine (R. Geiger, Chem. Ber., *101*, 3386 (1968)), or by catalytic reduction (G. Losse *et al.*, J. Prakt. Chem., *24*, 118 (1964)).

Among these methods of deformylation, the one where dilute hydrochloric acid is used is easy to operate as well as being inexpensive, and accordingly is a commerically excellent method. Deformylation with dilute hydrochloric acid is usually carried out as follows. For simple compounds such as N-formyl amino acids, the compound to be deformylated is heated with dilute hydrochloric acid (V. du Vigneaud *et al.*, J. Biol. Chem., *98*, 577 (1932)), while, for N-formyl peptides, the compound to be deformylated is allowed to stand in methanolic hydrochloric acid having a normality of not more than 0.5 at room temperature for 48 hours, whereby the peptide bond is prevented from splitting (J. C. Sheehan *et al.*, J. Am. Chem. Soc., *80*, 1158 (1958)).

However, in the case of N-formyl peptides (like For-α-APM) which have both an ester group and a free carboxyl group as well as the amino group protected with a formyl group, the ester and peptide bonds are also hydrolyzed if deformylation is carried out by acidic hydrolysis, while the free carboxyl group is esterified if deformylation is carried out by acidic-alcoholic hydrolysis. The latter hydrolysis is also commercially disadvantageous because a long period of time is required for the hydrolysis, e.g. a time as long as 48 hours (see JP—B—17727/1979, corresponding to US—A—3 879 372).

The inventors of the present invention have already proposed some improved methods of deformylation whereby the above difficulties are overcome to some extent, these improved methods being the hydroxylamine strong acid addition salt method (JP—A—68520/1976), corresponding to US—A—4 024 418) and the aqueous organic solvent-strong acid mixture method (JP—A—23001/1977), referred to hereinabove). These methods are, however, still commercially disadvantageous in that hydroxylamine is required for the former method and an organic solvent for the latter method.

The inventors have further proposed a commercially advantageous deformylation method devoid of the above-mentioned difficulties, in which method For-α-APM is heated in a 0.5 to 3N strong acid at 70 to 150°C whereby deformylation takes place selectively and is completed in a short period of time such as 15 seconds to 60 minutes, while side-reactions such as the hydrolysis of the ester or peptide bond are suppressed (see JP—A—131746/1982). However, this deformylation is not satisfactory, because the ester bond is still hydrolyzed to some degree.

In US—A—4 173 562, there is disclosed a process for preparing α-APM, which comprises contacting α - L - aspartyl - L - phenylalanine (for example α - L - aspartyl - L - phenylalanine formed by the removal of the formyl group from N - formyl - α - L - aspartyl - L - phenylalanine) with a reaction medium comprising water, methanol and hydrogen chloride to form a solid hydrochloride salt of α-APM, which salt is separated and converted to α-APM. This process, however, is not concerned with the problems arising in respect of the deformylation of For-α-APM.

The inventors have further investigated to find another commercially advantageous process of removing the N-formyl group from For-α-APM devoid of the abovementioned difficulties, and have found that, if the deformylation reaction is carried on by bringing For-α-APM into contact with a mixture of methanol and concentrated hydrochloric acid, preferably highly concentrated hydrochloric acid, at a temperature of from 0 to 40°C, while the α-APM formed is successively crystallized and eliminated from the reaction system as its sparingly soluble hydrochloride, side-reactions such as hydrolysis of the ester or peptide bond or esterification of the free carboxyl group are suppressed and high overall yields of α-APM are accordingly attained. A first embodiment of the present invention is based on this finding.

The process of this invention may be applied advantageously when α-APM is commercially produced from a precursor compound having a formyl group as an amino-protecting group. Processes for producing α-APM by the use of the formyl group as an amino-protecting group usually involve condensation between N - formyl - L - aspartic acid anhydride and L-phenylalanine methyl ester. However, this reaction gives not only For-α-APM, a precursor of the desired α-APM, but also N - formyl - β - L - aspartyl - L - phenylalanine methyl ester (referred to herein as "For-β-APM"), which is isomeric with For-α-APM.

An example of a conventional method for isolating the desired α-APM comprises treating a mixture of For-α-APM and For-β-APM with a mixture of methanol and dilute hydrochloric acid, neutralizing the resulting solution to precipitate α-APM, and isolating the α-APM from the remainder. According to this method, however, the above-mentioned side-reaction will take place because α-APM.HCl is not precipitated during the treatment, and when the α-APM is isolated after neutralization, part of it remains in the mother liquor in almost the same amount as the α-APM, because α-APM and β-APM (i.e. β - L - aspartyl - L - phenylalanine methyl ester) have almost the same solubility.

However, if the process of this invention is employed, α-APM.HCl, and, accordingly, also α-APM, may be efficiently obtained with purification effect, because, if a mixture of For-α-APM and For-β-APM is brought into contact with a mixture of methanol and higher concentrated hydrochloric acid, the formyl groups are removed to give α-APM and β-APM during the passage of time, while the α-APM formed is successively crystallized and eliminated from the reaction system as its sparingly soluble hydrochloride. Accordingly, the α-APM undergoes almost no side-reaction, while the β-APM is retained in the mother liquor. Incidentally, in connection with the fact that α-APM.HCl is very difficult to dissolve in hydrochloric acid, see US—A—3 798 207, corresponding to JP—B—41425/1974.

In connection with the deformylation conditions according to this invention, it has been found that, as the result of the inventor's study of the deformylation when carried out under various reaction conditions, the deformylation is remarkably affected by the concentrations of hydrochloric acid and methanol and by the reaction temperature.

According to the inventors' findings, when the hydrochloric acid concentration is too low, α-APM.HCl, is precipitated only in small amounts, while side-reactions such as hydrolysis of the ester bond occur. On the other hand, when the hydrochloric acid concentration is too high, the peptide bond and the ester group are hydrolyzed as rapidly as the formyl group is removed.

In connection with the reaction temperature, when it is too high, the peptide bond and the ester group are hydrolyzed as rapidly as the formyl group is removed, and also the α-APM.HCl is precipitated in small amounts.

In connection with the methanol concentration, when it is too low, the ester group undergoes hydrolysis, and, on the other hand, when it is too high, the free carboxyl group undergoes methyl-esterification, and also the α-APM.HCl is precipitated in small amounts.

In connection with the reaction time, too short a reaction time results in insufficient deformylation, and, on the other hand, too long a reaction time is disadvantageous from the commercial view point.

In consideration of the above, a hydrochloric acid concentration of from 2 to 12 normal, preferably 5 to 8 normal, a methanol concentration of from 5 to 60% by volume, preferably 10 to 30% by volume, based on the volume of the hydrochloric acid used, and a reaction temperature of from 0 to 40°C have been chosen as the preferred deformylation reaction conditions. Under these conditions, the deformylation is usually complete in a reaction time of from 1 to 5 days.

Examples 5, 6 and 11 to 17 below are examples of the first embodiment of this invention.

The inventors have found, as the result of their further investigation, that the above-described first embodiment of this invention, when modified in some respect, gives better effects, and second and third embodiments of this invention are based upon these findings.

According to this invention, the said second embodiment comprises a heating pretreatment wherein a mixture of For-α-APM, methanol and concentrated hydrochloric acid, preferably highly concentrated hydrochloric acid, is once heated to, and kept at, elevated temperature for a short time prior to deformylation of the For-α-APM by keeping the mixture at a temperature of from 0 to 40°C. From the practical point of view, the elevated temperature is from 50 to 100°C and the short time is a period of time not longer than 30 minutes.

By the heating treatment, the precipitation of α-APM.HCl may further be facilitated and time required for deformylation may be shortened from the above-mentioned period of 1 to 5 days to a period which is usually from 1 to 3 days.

The preferred conditions mentioned above in respect of the first embodiment apply also to the second embodiment.

Example 3 below is an example of the second embodiment of this invention.

The third embodiment of this invention is a modification of the second embodiment, wherein the same heating treatment is carried out at the same temperature and for the same time, but wherein concentrated hydrochloric acid, preferably highly concentrated hydrochloric acid, having a slightly reduced concentration is used and wherein concentrated hydrochloric acid, preferably highly concentrated hydrochloric acid, is further added sometime after the heating treatment. In this modification, the term "slightly reduced concentration" usually means a concentration of about 2 to 6 normal. Accordingly, the methanol is used in a slightly increased amount, usually in a concentration of from 10 to 70% by volume, preferably 30 to 55% by volume, based on the volume of the hydrochloric acid used. The hydrochloric acid is desirably used in an amount of from 0.5 to 3 moles, preferably about 1 to 1.3 moles, per mole of For-α-APM. The second-mentioned concentrated hydrochloric acid is preferably such an amount as to adjust the hydrochloric acid concentration in the reaction mixture to 2 to 12 normal, preferably 5 to 8 normal. Such further addition is, as has been mentioned, carried out sometime after the heating treatment. In greater detail, it is usually added after the heated reaction mixture has been cooled, though it may be added prior to, or during, the cooling.

The modification brings about further suppression of side-reactions, in addition to the above-mentioned facilitated precipitation of α-APM.HCl and shortened time required for deformylation, and, accordingly, higher yields of α-APM.HCl and thus free α-APM are obtained.

The α-APM hydrochloride obtained may be used as such as a sweetener (JP—A—13371/1974). It is, however, usually converted to free α-APM by neutralizing with the use of an alkali such as sodium carbonate in an aqueous solvent and collected as free α-APM crystals.

As is evident from the foregoing, this invention provides a commercially useful process for producing α-APM.HCl or α-APM, because, in accordance with this invention, the desired deformylated peptide may be produced, in the same or greater isolation yields than those achieved in accordance with the conventional prior art processes, by removing the formyl group from For-α-APM, the formed α-APM being crystallized as its sparingly soluble hydrochloride, with the use of only inexpensive reagents namely hydrochloric acid and methanol, and finally isolating the hydrochloride.

This invention will be illustrated by the following Examples.

Example 1

A mixture of 15 ml of methanol and 40 ml of 4N hydrochloric acid was heated to 60°C, and 48.3 g of For-α-APM were added to the heated mixture. The resulting mixture was kept at 60°C for 15 minutes, cooled rapidly to 25°C, mixed with 28 ml of concentrated hydrochloric acid, and stirred at 25°C for 2 days. The mixture was further stirred at 5°C for 3 hours.

The α-APM.HCl dihydrate crystals which precipitated were isolated from the remainder by filtering.

The α-APM content of the crystals was 36.1 g as measured by an amino acid analyser (Type 835, Hitachi Ltd.), which weight corresponds to a 81.8% yield based on the For-α-APM.

Incidentally, α-APM.HCl usually crystallizes from aqueous solution in dihydrate form (α-APM.HCl.2H₂O).

Example 2

To a mixed solvent of 10 ml of methanol and 17 ml of water, there was added a mixture of 32.2 g of For-α-APM and 8.1 g of For-β-APM. The resulting mixture was heated to 85°C, mixed with 10 ml of concentrated hydrochloric acid, kept at 85°C for 5 minutes, and cooled rapidly to 20°C. The cooled mixture, after being mixed with 20 ml of concentrated hydrochloric acid, was stirred at 20°C for 2 days and then further stirred at 5°C for 3 hours.

The resulting α-APM.HCl dihydrate crystals were isolated by filtering.

The α-APM content of the crystals was 23.4 g, which weight corresponds to a 79.5% yield based on the For-α-APM. No β-APM was detected.

Example 3

A mixed solvent of 15 ml of methanol and 65 ml of 7N hydrochloric acid was heated to 65°C, mixed with 48.3 g of For-α-APM, and kept at 65°C for 10 minutes. The resulting mixture was cooled rapidly to 20°C, and stirred at 20°C for 1 day and then at 5°C for 3 hours.

The precipitated crystals were collected by filtering.

The α-APM content of the crystals was 34.0 g, which weight corresponds to a 77.0% yield based on the For-α-APM.

Example 4

A mixed solvent of 15 ml of methanol, 25 ml of water and 14 ml of concentrated hydrochloric acid was heated to 60°C, and 48.3 g of For-α-APM were then added. The mixture was kept at 60°C for 15 minutes.

The mixture was then rapidly cooled to 20°C, 28 ml of concentrated hydrochloric acid were added and the mixture was stirred at 20°C for 2 days and then at 5°C for 3 hours.

The resulting crystals were collected by filtering.

The α-APM content of the crystals was 35.0 g. This weight corresponds to a 79.3% yield based on For-α-APM.

Example 5

To a mixed solvent of 15 ml of methanol, 25 ml of water and 42 ml of concentrated hydrochloric acid, there were added 48.3 g of For-α-APM. The mixture was stirred at 25°C for 4 days and then at 5°C for 3 hours.

The crystallized α-APM.HCl dihydrate was isolated by filtering.

The α-APM content of the crystals was 35.4 g. This weight corresponds to a 80.2% yield based on the For-α-APM.

Example 6

A mixture of 15 ml of methanol, 65 ml of 7N hydrochloric acid and 48.3 g of For-α-APM was stirred at 20°C for 4 days and then at 5°C for 3 hours.

The precipitated α-APM.HCl dihydrate crystals were isolated by filtering.

The isolated crystals were dissolved in 600 ml of water. The resulting solution, after being adjusted to a pH of 4.7, was mixed with sodium carbonate and allowed to stand overnight in a refrigerator.

The precipitated crystals were collected by filtering, washed with 100 ml of cold water, and dried.

The yield was 32.6 g of α-APM.1/2H$_2$O (71.6% yield based on the For-α-APM), having an optical rotation $[\alpha]_D^{20}$ of +15.6° (c=4, 15N formic acid). The purity of the crystals, measured with the amino acid analyser used in Example 1, was found to be over 98%.

Example 7

A mixed solvent of 15 ml of methanol, 25 ml of water and 14 ml of concentrated hydrochloric acid was heated to 70°C, and 48.3 g of For-α-APM were added to the heated solvent. The resulting mixture was kept at 70°C for 15 minutes. Thereafter, the mixture was rapidly cooled to 20°C, mixed with 14 ml of concentrated hydrochloric acid, and stirred at 20°C for 2 days and then at 5°C for 3 hours.

The resulting crystals were isolated by filtering.

The α-APM content of the crystals was 34.3 g, which corresponds to a 77.7% yield based on the For-α-APM.

Example 8

Example 4 was repeated, using 10 ml of methanol instead of 15 ml of methanol.

The yield of α-APM.HCl dihydrate was 82.3% based on the For-α-APM.

Example 9

Example 4 was repeated, using 20 ml of methanol instead of 15 ml of methanol.

The yield of α-APM.HCl.2H$_2$O was 73.6% based on the For-α-APM.

Example 10

A mixture of 18 ml of methanol, 30 ml of water and 17 ml of concentrated hydrochloric acid was heated to 70°C, and 48.3 g of For-α-APM were added. The resulting mixture was kept at 70°C for 10 minutes, rapidly cooled to 20°C and, after the addition of 24 ml of concentrated hydrochloric acid, stirred at 20°C for 2 days and then at 5°C for 3 hours.

The precipitated α-APM.HCl.2H$_2$O crystals were collected by filtering.

The α-APM content of the collected crystals was 33.1 g, corresponding to a 75.0% yield based on the For-α-APM.

Examples 11 to 17

These Examples were carried out under the conditions given in the following Table, the other conditions being the same as in Example 5. The results are also listed in the Table.

TABLE

| Example | Methanol (ml) | Water (ml) | Conc. Hydrochloric acid (ml) | Reaction temp. (°C) | Reaction time (day) | Isolation yield of α-APM.HCL.2H$_2$O (based on For-α-APM) (%) |
|---------|---------------|------------|------------------------------|---------------------|---------------------|------------------------------------------------------------------|
| 11 | 10 | 25 | 42 | 25 | 4 | 81.5 |
| 12 | 20 | 36 | 50 | 20 | 4 | 78.2 |
| 13 | 15 | 25 | 25 | 25 | 2 | 75.6 |
| 14 | 20 | 25 | 42 | 20 | 3 | 75.3 |
| 15 | 15 | 25 | 42 | 20 | 1 | 70.1 |
| 16 | 15 | 25 | 42 | 35 | 3 | 75.8 |
| 17 | 15 | 25 | 42 | 5 | 4 | 79.9 |

## Claims

1. A process for producing α - L - aspartyl - L - phenylalanine methyl ester or its hydrochloride, which comprises bringing N - formyl - α - L - aspartyl - L - phenylalanine methyl ester into contact with a mixture of methanol and concentrated hydrochloric acid at a temperature of from 0 to 40°C whereby the amino-protecting formyl group is removed while the α - L - aspartyl - L - phenylalanine methyl ester formed is crystallized as its sparingly soluble hydrochloride; isolating the hydrochloride crystals; and, if desired, converting the hydrochloride to free α - L - aspartyl - L - phenylalanine methyl ester.

2. A process for producing α - L - aspartyl - L - phenylalanine methyl ester or its hydrochloride, which comprises maintaining a mixture of N - formyl - α - L - aspartyl - L - phenylalanine methyl ester, methanol and concentrated hydrochloric acid at an elevated temperature; cooling the mixture to a temperature of from 0 to 40°C; maintaining the mixing at a temperature of from 0 to 40°C whereby the amino-protecting formyl group is removed while the α - L - aspartyl - L - phenylalanine methyl ester formed is crystallized as its sparingly soluble hydrochloride; isolating the hydrochloride crystals; and, if desired, converting the hydrochloride to free α - L - aspartyl - L - phenylalanine methyl ester.

3. A process for producing α - L - aspartyl - L - phenylalanine methyl ester or its hydrochloride, which comprises maintaining a mixture of N - formyl - α - L - aspartyl - L - phenylalanine methyl ester, methanol and concentrated hydrochloric acid at an elevated temperature; adding concentrated hydrochloric acid to the mixture; maintaining the resulting mixture at a temperature of from 0 to 40°C whereby the amino-protecting formyl group is removed while the α - L - aspartyl - L - phenylalanine methyl ester formed is crystallized as its sparingly soluble hydrochloride; isolating the hydrochloride crystals; and, if desired, converting the hydrochloride to free α - L - aspartyl - L - phenylalanine methyl ester.

4. A process according to claim 1 or 2, wherein the hydrochloric acid has a normality of from 2 to 12.

5. A process according to claim 1, 2 or 4, wherein the methanol concentration of the mixture is from 5 to 60% by volume based upon the volume of hydrochloric acid.

6. A process according to claim 3, wherein the mixture is maintained at a temperature of from 50 to 100°C for a time not longer than 30 minutes.

7. A process according to claim 3 or 6, wherein the first-mentioned hydrochloric acid has a normality of from 2 to 6.

8. A process according to claim 3, 6 or 7, wherein the second-mentioned hydrochloric acid has a normality such that the resulting mixture has a hydrochloric acid normality of from 2 to 12.

9. A process according to claim 3, 6, 7 or 8, wherein the methanol concentration of the mixture is from 10 to 70% by volume based upon the volume of hydrochloric acid.

10. α - L - aspartyl - L - phenylalanine methyl ester or its hydrochloride, when produced by a process according to any of claims 1 to 9.

**Revendications**

1. Procédé pour la production d'ester méthylique d'α - L - aspartyl - L - phénylalanine ou de son chlorhydrate, caractérisé en ce qu'il consiste à mettre en contact l'ester méthylique de N - formyl - α - L - aspartyl - L - phénylalanine avec un mélange de méthanol et d'acide chlorhydrique concentré à une température de 0 à 40°C, de sorte que le groupe amino-protecteur formyle est éliminé tandis que l'ester méthylique d'α - L - aspartyl - L - phénylalanine formé est cristallisé sous forme de son chlorhydrate peu soluble; à isoler les cristaux de chlorhydrate; et, si on le désire, à convertir le chlorhydrate en ester méthylique d'α - L - aspartyl - L - phénylalanine libre.

2. Procédé pour la production d'ester méthylique d'α - L - aspartyl - L - phénylalanine ou de son chlorhydrate, caractérisé en ce qu'il consiste à maintenir un mélange de N - formyl - α - L - aspartyl - L - phénylalanine, de méthanol et d'acide chlorhydrique concentré à une température élevée; à refroidir le mélange à une température de 0 à 40°C; à maintenir le mélange à une température de 0 à 40°C de manière à éliminer le groupement formyle amino-protecteur, tandis que l'ester méthylique d'α - L - aspartyl - L - phénylalanine formé est cristallisé sous forme de son chlorhydrate peu soluble; à isoler les cristaux de chlorhydrate; et, si on le désire, à convertir le chlorhydrate en ester méthylique d'α - L - aspartyl - L - phénylalanine libre.

3. Procédé pour la production d'ester méthylique d'α - L - aspartyl - L - phénylalanine ou de son chlorhydrate, caractérisé en ce qu'il consiste à maintenir un mélange de N - formyl - α - L - aspartyl - L - phénylalanine, de méthanol et d'acide chlorhydrique concentré à une température élevée; à ajouter au mélange de l'acide chlorhydrique concentré; à maintenir le mélange résultant à une température de 0 à 40°C, de manière à éliminer le groupe formyle amino-protecteur, tandis que l'ester méthylique d'α - L - aspartyl - L - phénylalanine formé est cristallisé sous forme de son chlorhydrate peu soluble; à isoler les cristaux de chlorhydrate; et, si on le désire, à convertir le chlorhydrate en ester méthylique d'α - L - aspartyl - L - phénylalanine libre.

4. Procédé selon la revendication 1 ou 2, caractérisé en ce que l'acide chlorhydrique a une normalité de 2 à 12.

5. Procédé selon la revendication 1, 2 ou 4, caractérisé en ce que la concentration du méthanol dans le mélange est de 5 à 60% en volume, par rapport au volume d'acide chlorhydrique.

6. Procédé selon la revendication 3, caractérisé en ce que le mélange est maintenu à une température de 50 à 100°C pendant une durée de pas plus de 30 min.

7. Procédé selon la revendication 3 ou 6, caractérisé en ce que le premier acide chlorhydrique mentionné a une normalité de 2 à 6.

8. Procédé selon la revendication 3, 6 ou 7, caractérisé en ce que le second acide chlorhydrique mentionné a une normalité telle que le mélange résultant ait une normalité d'acide chlorhydrique de 2 à 12.

9. Procédé selon la revendication 3, 6, 7 ou 8, caractérisé en ce que la concentration de méthanol dans le mélange est de 10 à 70% en volume, par rapport au volume d'acide chlorhydrique.

10. Ester méthylique d'α - L - aspartyl - L - phénylalanine ou son chlorhydrate, caractérisé en ce qu'il est produit par un procédé selon l'une quelconque des revendications 1 à 9.

## Patentansprüche

1. Verfahren zur Herstelling von α - L - Aspartyl - L - phenylalanin - methylester oder dessen Hydrochlorid bei dem N - Formyl - α - L - aspartyl - L - phenylalanin - methylester bei einer Temperatur von 0 bis 40°C mit einem Gemisch aus Methanol und konzentrierter Chlorwasserstoffsäure in Berührung gebracht wird, wodurch die als Schutzgruppe für die Aminogruppe vorliegende Formylgruppe entfernt wird, während der gebildete α - L - Aspartyl - L - phenylalanin - methylester in Form seines schwerlöslichen Hydrochlorids kristallisiert, das kristalline Hydrochlorid isoliert wird und gewünschtenfalls das Hydrochlorid in freien α - L - Aspartyl - L - phenylalanin - methylester umgewandelt wird.

2. Verfahren zur Herstellung von α - L - Aspartyl - L - phenylalanine - methylester oder dessen Hydrochlorid, bei dem ein Gemisch aus N - Formyl - α - L - aspartyl - L - phenylalanin - methylester, Methanol und konzentrierter Chlorwasserstoffsäure bei erhöhter Temperatur gehalten wird, das Gemisch auf eine Temperatur von 0 bis 40°C abgekühlt wird, das Gemisch bei einer Temperatur von 0 bis 40°C gehalten wird, wobei die als Schutzgruppe für die Aminogruppe vorliegende Formylgruppe entfernt wird, während der gebildete α - L - Aspartyl - L - phenylalanin - methylester in Form seines schwerlöslichen Hydrochlorids kristallisiert, dans kristalline Hydrochlorid isoliert wird und gewünschtenfalls das Hydrochlorid in freien α - L - Aspartyl - L - phenylalanin - methylester umgewandelt wird.

3. Verfahren zur Herstellung von α - L - Aspartyl - L - phenylalanin - methylester oder dessen Hydrochlorid, bei dem ein Gemisch aus N - Formyl - α - L - aspartyl - L - phenylalanin - methylester, Methanol und konzentrierter Chlorwasserstoffsäure bei erhöhter Temperatur gehalten wird, zu dem Gemisch konzentrierte Chlorwasserstoffsäure zugefügt wird, das gebildete Gemisch bei einer Temperatur von 0 bis 40°C gehalten wird, wodurch die als Schutzgruppe für die Aminogruppe vorliegende Formylgruppe entfernt wird, während der gebildete α - L - Aspartyl - L - phenylalanin - methylester in Form seines schwerlöslichen Hydrochlorids kristallisiert, das kristalline Hydrochlorid isoliert wird und gewünschtenfalls das Hydrochlorid in freien α - L - Aspartyl - L - phenylalanin - methylester umgewandelt wird.

4. Verfahren nach Anspruch 1 oder 2, bei dem die Chlorwasserstoffsäure 2- bis 12-normal ist.

5. Verfahren nach Anspruch 1, 2 oder 4, bei dem die Methanol-Konzentration des Gemisches 5 bis 60 Volum-%, bezogen auf das Volumen der Chlorwasserstoffsäure, beträgt.

6. Verfahren nach Anspruch 3, bei dem das Gemisch bei einer Temperatur von 50 bis 100°C während einer Dauer von nicht mehr als 30 Minuten gehalten wird.

7. Verfahren nach Anspruch 3 oder 6, bei dem dei zuerst erwähnte Chlorwasserstoffsäure 2- bis 6-normal ist.

8. Verfahren nach Anspruch 3, 6 oder 7, bei dem die an zweiter Stelle erwähnte Chlorwasserstoffsäure einen solchen Wert der Normalität hat, daß das resultierende Gemisch eine Normalität der Chlorwasserstoffsäure von 2 bis 12 aufweist.

9. Verfahren nach Anspruch 3, 6, 7 oder 8, bei dem die Methanol-Konzentration des Gemisches 10 bis 70 Volum-%, bezogen auf das Volumen der Chlorwasserstoffsäure, beträgt.